(19) Europäisches Patentamt
European Patent Office
Office européen des brevets

(11) **EP 3 446 626 A1**

(12) **EUROPEAN PATENT APPLICATION**
published in accordance with Art. 153(4) EPC

(43) Date of publication:
**27.02.2019 Bulletin 2019/09**

(51) Int Cl.:
**A61B 5/00** (2006.01)   **A61B 5/103** (2006.01)
**G01N 21/33** (2006.01)

(21) Application number: **17785445.2**

(22) Date of filing: **20.04.2017**

(86) International application number:
**PCT/CN2017/081187**

(87) International publication number:
**WO 2017/181961 (26.10.2017 Gazette 2017/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.04.2016 CN 201610246311**

(71) Applicant: **Chen, Wei-Yu**
**Taipei City, Taiwan 10441 (CN)**

(72) Inventor: **Chen, Wei-Yu**
**Taipei City, Taiwan 10441 (CN)**

(74) Representative: **Zimmermann, Tankred Klaus**
**Schoppe, Zimmermann, Stöckeler Zinkler, Schenk & Partner mbB**
**Patentanwälte**
**Radlkoferstrasse 2**
**81373 München (DE)**

(54) **DETECTION METHOD AND DETECTION DEVICE FOR INDIVIDUAL MINIMAL ERYTHEMA DOSE**

(57)   A method for detecting a minimal erythema dose includes: detecting a pigment index of a body to obtain a predetermined minimal erythema dose and a predetermined erythema generation index; obtaining a dose value of a UV light source; detecting a redness situation of a skin area of the body by a first time to obtain a first redness index; applying a UV absorption material having an SPF on the skin area; irradiating a UV light from the UV light source on the skin area and detecting a redness situation of the skin area by a second time to obtain a second redness index; when a difference between the first and second redness indexes is larger than the predetermined erythema generation index, obtaining a time value between the first and second detecting steps; and obtaining the minimal erythema dose of the body according the dose value, SPF and time value. The invention can know the dose of the actual UV light that the skin of the body can resist by correcting the minimum erythema dose of the body.

FIG. 1A

Flowchart steps:
- detecting a pigment index of a body — S10
- obtaining a predetermined minimal erythema dose and a predetermined erythema generation index according to the pigment index — S20
- obtaining a dose value of a UV light source — S30
- detecting a redness situation of a skin area of the body by a first time to obtain a first redness index — S40
- applying a UV absorption material, which has an SPF (sun protection factor), on the skin area — S50
- irradiating a UV light from the UV light source on the skin area and then detecting a redness situation of the skin area of the body by a second time to obtain a second redness index — S60
- if a difference between the first redness index and the second redness index is larger than or equal to the predetermined erythema generation index, obtaining a first time value, which is the time between the first and second detecting steps — S70
- obtaining the minimal erythema dose of the body according the dose value, the SPF and the first time value — S80

EP 3 446 626 A1

**Description**

**BACKGROUND OF THE INVENTION**

**Field of Invention**

The present invention relates to a detecting method and apparatus, and in particular, to a detecting method and apparatus for skin.

**Related Art**

The recent researches indicates that the exposure under the sun may danger the human body, such as causing the skin aging, wrinkles, and skin cancer. In particular, since the UV light increases due to the thinner of the ozone layer, the skin will be easily burned under the sun. In fact, the most harmful part of the UV light is the UVB light (middle-wave). If the skin is irradiated by excessive UVB light, the skin may be burned and inflamed, which will lead to the vasodilation and skin redness.

In general, the proper sunscreen product can be applied to the skin to avoid the excessive UV light irradiating on the skin. The sunscreen products include a chemical protection mechanism and a physical protection mechanism. The chemical protection mechanism utilizes the specific chemical compound to absorb the UV light for protecting the skin. The physical protection mechanism is to reflect or refract the UV light to achieve the desired protection effect. The protection ability of the sunscreen products is classified by the SPF (sun protection factor). The sun protection factor is calculated based on the ratio of the total time that will not cause sunburn to the skin which with/without wearing sunscreen products after exposure to the same UV light. For example, if a person wears a sunscreen with an SPF of 4, assuming sunscreen is applied evenly at a thick dosage of 2 milligrams per square centimeter (mg/cm2), the length of time it takes for him or her to suffer a burn will extend four times. Thus, if a person develops a sunburn in 1 hour when not wearing a sunscreen, the same person in the same intensity of sunlight will avoid sunburn for 4 hours if wearing a sunscreen with an SPF of 4.

In practice, although the user applies the sunscreen product with the suggested SPF, he or she still usually has sunburns. This is because the SPF suggestion is created based on a general situation (body) and regardless the differences between different bodies. In more detailed, the general situation assumes that the users have the same MED (minimal erythema dose), which is the UV light irradiation time or dose for causing the minimal erythema that can be seen by human eyes. In fact, MED is various in different bodies. Moreover, MED is various in different color skins and different skin parts. Thus, the SPF suggestion created based on the general situation is not reliable. In some cases, the user will apply the sunscreen product again when the previous applied sunscreen product is still protection function, which will cause the waste of the sunscreen product. In some cases, the previous applied sunscreen product has lost the protection function, but the user doesn't know to apply the next sunscreen product, which may cause the sunburns and skin cancer.

Therefore, it is desired to provide a detecting method that can provide the real MED of the individual body so as to accurately calculate the maximum UV light exposure time and maximum UV dose acceptable for the skin of the individual body (the exposure time and dose have positive correlation). Besides, the user can precisely determine the time for applying the next sunscreen product so as to avoid the undesired waste.

**SUMMARY OF THE INVENTION**

In view of the foregoing, it is an objective of the present to provide a method and apparatus for detecting the MED (minimal erythema dose) of a body, which can obtain the actual MED of each user, thereby preventing the damages or diseases of skin (e.g. sunburns or melanin deposition) caused by incorrect protection time of the sunscreen product determined based on the predetermined MED. Besides, the present invention can obtain the actual MED of each user for assisting to evaluate the protection time or maximum UV dose after applying the sunscreen product, so that the user can perfectly apply the sunscreen product again to achieve the most economic usage of the sunscreen product.

To achieve the above objective, the present invention discloses a method for detecting a minimal erythema dose of a body. The method includes the following steps of: detecting a pigment index of the body; obtaining a predetermined minimal erythema dose and a predetermined erythema generation index according to the pigment index; obtaining a dose value of a UV light source; detecting a redness situation of a skin area of the body by a first time to obtain a first redness index; applying a UV absorption material, which has an SPF (sun protection factor), on the skin area; irradiating a UV light from the UV light source on the skin area and then detecting a redness situation of the skin area of the body by a second time to obtain a second redness index; if a difference between the first redness index and the second redness index is larger than or equal to the predetermined erythema generation index, obtaining a first time value, which is the time between the first and second detecting steps; and obtaining the minimal erythema dose of the body according the dose value, the SPF and the first time value.

In one embodiment, the method further includes the following steps of: obtaining a predetermined detecting time value according to the predetermined minimal erythema dose, the dose value and the SPF; obtaining a difference between the predetermined detecting time value and the first time value; and modifying the predetermined minimal erythema dose to obtain the minimal erythema dose according to the difference between the first redness index and the second redness index, the difference between the predetermined detecting time value and the first time value, and the predetermined erythema generation index.

In one embodiment, the predetermined minimal erythema dose and/or the predetermined erythema generation index has a positive correlation to the pigment index.

In one embodiment, the method further includes the following steps of: if the difference between the first redness index and the second redness index is smaller than the predetermined erythema generation index, continuously irradiating the UV light from the UV light source on the skin area and then detecting a redness situation of the skin area of the body by a third time to obtain a third redness index; if a difference between the first redness index and the third redness index is larger than or equal to the predetermined erythema generation index, obtaining a second time value, which is the time between the first and third detecting steps; and obtaining the minimal erythema dose of the body according the dose value, the SPF and the second time value.

In one embodiment, the UV absorption material is a sunscreen lotion or a foundation product.

To achieve the above objective, the present invention also discloses a detecting apparatus for detecting a minimal erythema dose of a body. The detecting apparatus includes a UV detection device, a light sensing device, one or more processing units, and a storage unit. The one or more processing units signally connect to the UV detection device and the light sensing device, and the storage unit signally connects to the one or more processing units and stores one or more programs. When the one or more processing units execute the one or more programs, the one or more processing units perform the following steps of: obtaining a pigment index of the body by the light sensing device; obtaining a predetermined minimal erythema dose and a predetermined erythema generation index according to the pigment index; obtaining a dose value of a UV light source by the UV detection device; detecting a redness situation of a skin area of the body by a first time to obtain a first redness index by the light sensing device; obtaining an SPF (sun protection factor) of a UV absorption material, which is applied on the skin area; after the UV light source irradiates a UV light on the skin area, detecting a redness situation of the skin area of the body by a second time to obtain a second redness index by the light sensing device; if a difference between the first redness index and the second redness index is larger than or equal to the predetermined erythema generation index, obtaining a first time value, which is the time between the first and second detecting steps; and obtaining the minimal erythema dose of the body according the dose value, the SPF and the first time value.

In one embodiment, the one or more processing units further perform the following steps of: obtaining a predetermined detecting time value according to the predetermined minimal erythema dose, the dose value and the SPF; obtaining a difference between the predetermined detecting time value and the first time value; and modifying the predetermined minimal erythema dose to obtain the minimal erythema dose according to the difference between the first redness index and the second redness index, the difference between the predetermined detecting time value and the first time value, and the predetermined erythema generation index.

In one embodiment, the predetermined minimal erythema dose and/or the predetermined erythema generation index has a positive correlation to the pigment index.

In one embodiment, the one or more processing units further perform the following steps of: if the difference between the first redness index and the second redness index is smaller than the predetermined erythema generation index, after the UV light source continuously irradiates the UV light on the skin area, detecting a redness situation of the skin area of the body by a third time to obtain a third redness index; if a difference between the first redness index and the third redness index is larger than or equal to the predetermined erythema generation index, obtaining a second time value, which is the time between the first and third detecting steps; and obtaining the minimal erythema dose of the body according the dose value, the SPF and the second time value.

In one embodiment, the UV absorption material is a sunscreen lotion or a foundation product.

In one embodiment, the detecting apparatus is a portable detecting apparatus.

In one embodiment, the detecting apparatus further includes an NIR (near infrared) light source for emitting an NIR light. The light sensing device detects the NIR light scattered from the skin area so as to obtain the first redness index or the second redness index.

In one embodiment, the detecting apparatus further includes a red light source for emitting a red light, and the light sensing device detects the red light scattered from the skin area so as to obtain the first redness index or the second redness index.

In one embodiment, the detecting apparatus further includes a green light source for emitting a green light, and the light sensing device detects the green light reflected from the skin area so as to obtain the pigment index.

In one embodiment, the detecting apparatus includes two green light sources.

In one embodiment, the positions of the two green light sources are perpendicular to each other.

In one embodiment, the NIR light source, the red light source and the two green light sources are disposed around the light sensing device.

As mentioned above, the method and apparatus of the present invention can calibrate the MED of a body by detecting the UV dose in the environment, measuring the variation of the redness of skin and the time before and after receiving the UV light, and obtaining the SPF of the UV absorption material. The detecting apparatus of the invention is easily operated to perform the above steps. After obtaining the actual MED of the user, the maximum UV dose or the maximum exposure time under the sunlight after applying the UV absorption material can be calculated according to the SPF of the UV absorption material and the actual MED. Besides, the detecting apparatus of the invention can further prompt the user to apply the UV absorption material again at the right time so as to prevent the damage of skin (e.g. sunburns).

## BRIEF DESCRIPTION OF THE DRAWINGS

FIGS. 1A to 1C are flow charts showing the steps of the method for detecting a minimal erythema dose of a body according to a preferred embodiment of the invention;

FIG. 2A is a block diagram of a detecting apparatus according to a preferred embodiment of the invention;

FIG. 2B is a schematic diagram showing the configuration of the detecting apparatus according to the preferred embodiment of the invention;

FIG. 2C is a schematic diagram showing the configuration of a detection light emitting unit and a light sensing device of the detecting apparatus according to the preferred embodiment of the invention;

FIG. 3A is a schematic diagram showing the operation status of the detecting apparatus according to the preferred embodiment of the invention; and

FIG. 3B is a schematic diagram showing the detecting procedure of the detecting apparatus according to the preferred embodiment of the invention.

## DETAILED DESCRIPTION OF THE INVENTION

The present invention will be apparent from the following detailed description, which proceeds with reference to the accompanying drawings, wherein the same references relate to the same elements.

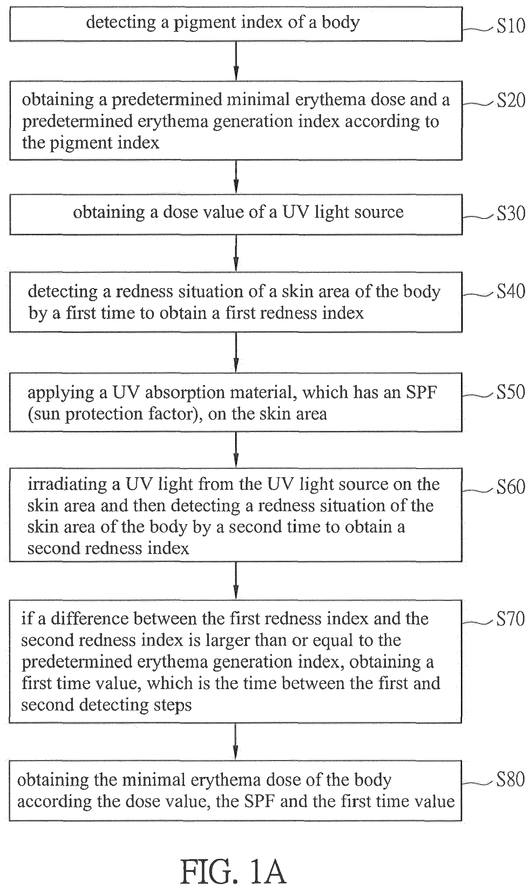

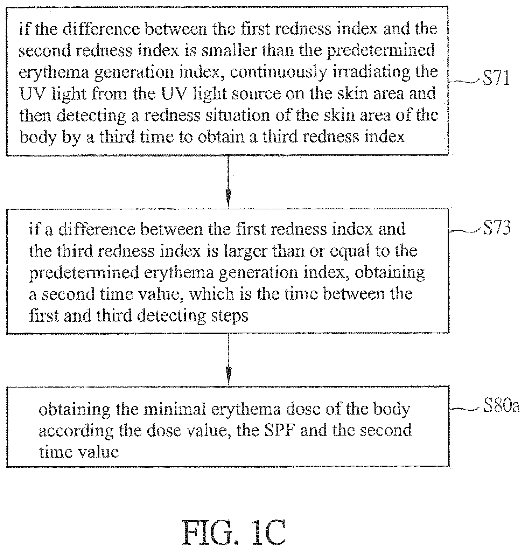

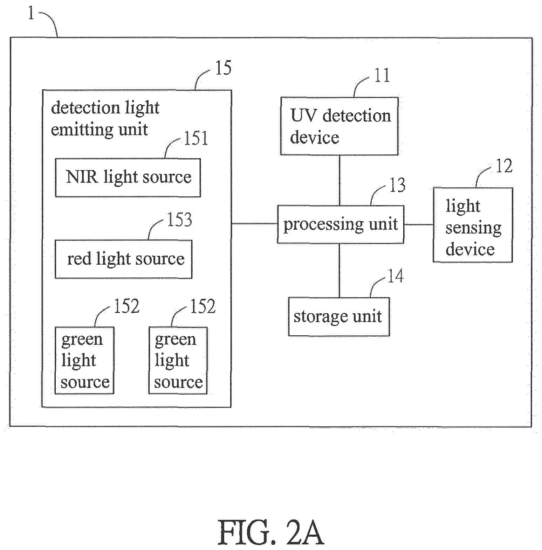

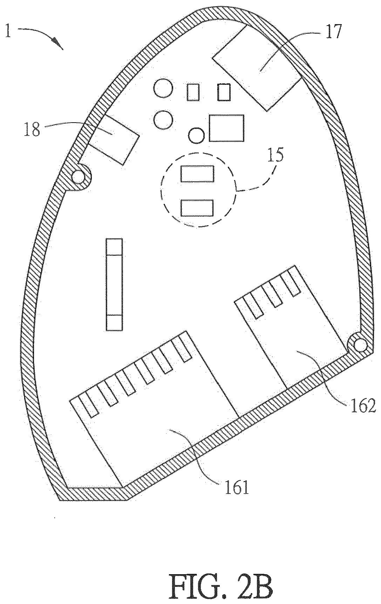

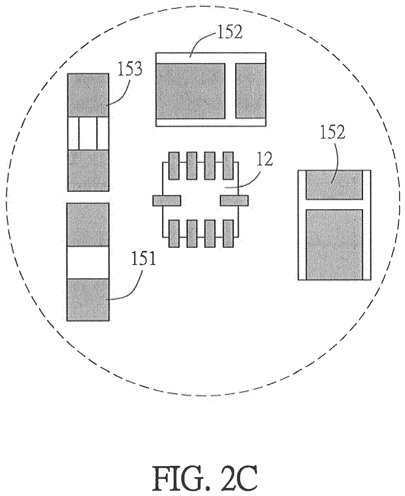

FIGS. 1A to 1C are flow charts showing the steps of the method for detecting a minimal erythema dose of a body according to a preferred embodiment of the invention. FIG. 2A is a block diagram of a detecting apparatus according to a preferred embodiment of the invention. FIG. 2B is a schematic diagram showing the configuration of the detecting apparatus according to the preferred embodiment of the invention. FIG. 2C is a schematic diagram showing the configuration of a detection light emitting unit and a light sensing device of the detecting apparatus according to the preferred embodiment of the invention. Referring to FIGS. 1A to 1C and 2A to 2C, the method for detecting a minimal erythema dose of a body is performed by a detecting apparatus 1, but this invention is not limited thereto. The priorities of the step 10 and step 40 are not limited. That is, the step 10 can be performed before or after the step 40. The order of the steps in this embodiment is for an illustration only and is not to limit the invention.

The detecting apparatus 1 includes a UV detection device 11, a light sensing device 12, a processing unit 13, a storage unit 14 and a detection light emitting unit 15. The processing unit 13 signally connects to the UV detection device 11, the light sensing device 12, the storage unit 14 and the detection light emitting unit 15. The processing unit 13 accesses the programs and data from the storage unit 14 and then calculates according to the accessed programs so as to control other devices or units. The detection light emitting unit 15 is controlled by the processing unit 13 to emit the light of the corresponding wavelength in different situations for performing the desired detection procedure. Preferably, the detection apparatus 1 is a portable apparatus or a wearable apparatus, which is different from the conventional large-sized skin detection instrument, so the detection apparatus 1 of the invention can be easily carried for detecting the intensity of the local UV light in real time. In other words, since the conventional large-sized skin detection instrument can't retrieve the intensity of the local UV light in real time, it is hard to carry out the function of the invention to detect the minimal erythema dose (MED) of a body. In addition, the detecting apparatus 1 is also different from the conventional simulated skin detecting apparatus carried out by using a smart phone to execute a corresponding mobile application (APP). The conventional simulated skin detecting apparatus uses the camera module of the smart phone to capture the skin photo for analyzing. However, this approach can't analyze the skin by lights of different wavelengths (e.g. detecting melanin of a body).

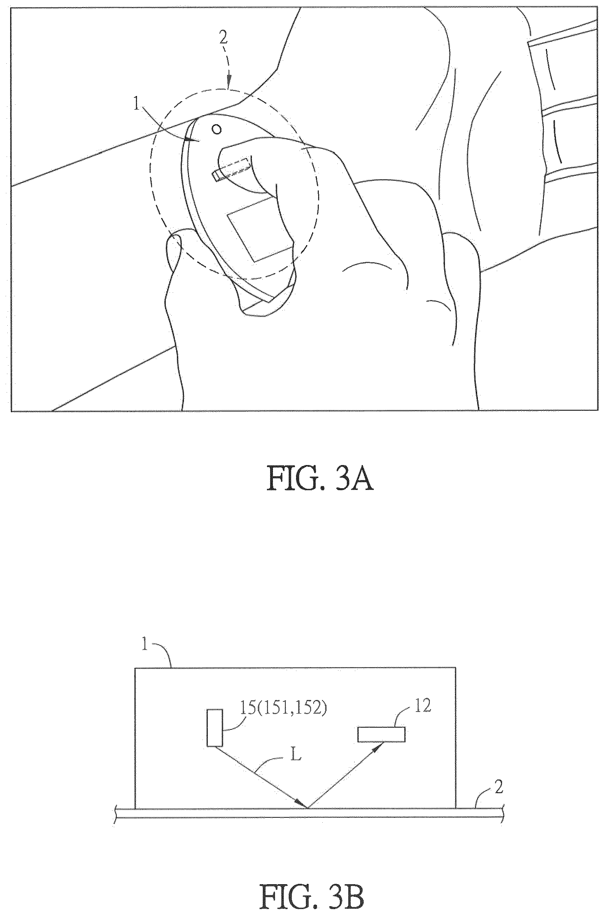

The step S10 is to detect a pigment index of a body. FIG. 3A is a schematic diagram showing the operation status of the detecting apparatus according to the preferred embodiment of the invention, and FIG. 3B is a schematic diagram showing the detecting procedure of the detecting apparatus according to the preferred embodiment of the invention. As shown in FIG. 3A, the detecting apparatus 1 is attached on the skin area 2 of a user. In the detecting procedure, as shown in FIG. 3B, the detection light emitting unit 15 emits a light L toward the skin area 2, and then the light L is scattered by the skin area 2 and received by the light sensing device 12. Afterwards, the received light signal is transmitted from the light sensing device 12 to the processing unit 13 for following calculation to obtain a pigment index of the skin area 2. Herein, the pigment index indicates the dark color of the skin area, which is mostly determined by the melanin amount. Accordingly, the pigment index can also be detected by using an image sensor (e.g. CMOS or camera) to obtain the image or photo of the skin area 2 and then analyzing the image or photo. Alternatively, it is also possible to directly detect the melanin amount of the skin area 2 for representing the pigment index of the skin area 2.

Referring to FIGS. 2B and 2C, the detecting apparatus 1 further includes two connectors 161 and 162, an USB port 17, and a humidity sensing unit 18. To make the drawings more clear, the UV detection device 11, the light sensing device 12, the processing unit 13 and the storage unit 14 are not shown in FIG. 2B. The detection light emitting unit 15 includes an NIR light source 151, a red light source 153 and a green light source 152. The green light source 152 emits a green light for obtaining the pigment index of the skin area 2 of the body. The NIR light source 151 and the red light source 153 emit an NIR light and a red light, respectively, for detecting the redness of the skin area 2. In practice, the NIR light source 151, the red light source 153 and the green light source 152 can be all made by LEDs (light-emitting diodes).

As shown in FIG. 2C, the light sensing device 12 is preferably disposed at the same area as the detection light emitting unit 15, and the NIR light source 151, the red light source 153 and the green light source 152 are disposed around the light sensing device 12. Since the cells in the skin area are arranged in a two-dimensional direction, the detection light emitting unit 15 preferably includes two green light sources 152, which are disposed perpendicular to each other. This configuration can improve the absorption rate of the scattered light signals in a single light direction, thereby averaging the absorption amount of the skin area so as to obtain a more precise pigment index of the skin area. In this embodiment, the wavelength of the green light ranges between 500nm and 570nm, and is preferably 519nm. The wavelength of the NIR light ranges between 750nm and 1400nm, and is preferably 940nm. The wavelength of the red light ranges between 620nm and 750nm, and is preferably 660nm.

Preferably, the body portion to be detected in the step S10 is different from the body portions to be detected in the following steps (e.g. steps S40 and S60). For example, it is preferred to select a portion of the skin that is seldom exposed to sunlight (e.g. the inner side of arm or buttock) for the step S10 to obtain the pigment index of the body. On the contrary, it is preferred to select a portion of the skin that is usually exposed to sunlight (e.g. the outer side of arm or face) to detect the redness index. The above detection rule is based on that the skin of the inner side of arm or buttock is seldom exposed to sunlight, so that the pigment index of this portion is much closer to the original skin of the body, which can minimize the detection deviation and thus prevent the mismeasurement of the minimal erythema dose of the body. Besides, the skin of the outer side of arm or face is suitable to be detected for determining whether body has sunburn or not.

The step S20 is to obtain a predetermined minimal erythema dose and a predetermined erythema generation index according to the pigment index. In this embodiment, the detecting apparatus 1 has a look-up table containing the pigment indexes, predetermined minimal erythema doses and predetermined erythema generation indexes. The look-up table can be stored in the storage unit 14 or the firmware of the detecting apparatus 1. The following table shows an example of the look-up table. In this embodiment, the term "predetermined erythema generation index" is a maximum variation value of the skin redness situation without having sunburn under the irradiation of the sunlight (containing UV light) or UV light.

Table 1: The look-up table containing the pigment indexes, predetermined minimal erythema doses and predetermined erythema generation indexes

| pigment index (unit) | level | predetermined minimal erythema dose (unit: minutes) | predetermined erythema generation index |
|---|---|---|---|
| 9 or less | 0 | 10 | 1 |
| 10-19 | 1 | 15 | 3 |
| 20-29 | 2 | 20 | 5 |

(continued)

| pigment index (unit) | level | predetermined minimal erythema dose (unit: minutes) | predetermined erythema generation index |
|---|---|---|---|
| 30-39 | 3 | 25 | 7 |
| 40-49 | 4 | 30 | 10 |
| 50 or more | 5 | 45 | 15 |

As shown in the above table, the pigment index is in positive correlation with the predetermined minimal erythema dose and/or the predetermined erythema generation index. In more details, when the skin has higher pigment index, the corresponding predetermined minimal erythema doses and/or predetermined erythema generation index is also higher. Accordingly, the detecting apparatus 1 can obtain the predetermined minimal erythema doses and predetermined erythema generation index by the look-up table stored in the processing unit 13 with the pigment index of the skin area 2 transmitted from the light sensing unit 12 to the processing unit 13. Of course, in other embodiments, the desired predetermined minimal erythema doses and predetermined erythema generation index can also be obtained by calculations instead of the look-up table, which can reduce the occupied memory capacity.

In addition, the method of this embodiment further include a melanin grouping step for establishing the above table. In this step, the amount and distribution of the melanin are detected in advance, and the bodies are divided into several groups according to the standards of the database. The average minimal erythema doses and erythema generation index of each group are defined as the predetermined minimal erythema doses and predetermined erythema generation index. Afterwards, the above table 1 can be established based on the obtained predetermined minimal erythema doses and predetermined erythema generation index. The entire database can be established by collecting the pigment indexes of all kinds of bodies and skins, and the standards for grouping can also be defined accordingly. Accordingly to this steps, since the samples of the database can continuously increase, the standards, predetermined minimal erythema doses and predetermined erythema generation index can be adjusted accordingly. This feature is benefit to increase the accurate of the following calculated minimal erythema doses.

The step S30 is to obtain a dose value of a UV light source. In practice, the step S30 can detect the UV light dose of the sunlight in the environment where the user stays, and the obtained UV light dose can be used to calculate and evaluate the maximum exposure time in this environment for the user. In this embodiment, the portable or wearable detecting apparatus 1 has a UV detection device for detecting the UV light source in the environment where the user stays. Accordingly, the UV light intensity or dose of this environment can be detected, and the detected value is much better than the data retrieved through Internet. In addition, the UV light source may be the UV lamp used in the medical instrument or tanning machine. This application can prevent the undesired hurt or accident while using the medical instrument or tanning machine.

The step S40 is to detect a redness situation of a skin area of the body by a first time to obtain a first redness index. The first redness index represents the redness index of the skin of the user before exposing to the sunlight (UV light source), and it can be the base for calculating and/calibrating the minimal erythema dose of the user.

Referring to FIG. 3, the step S40 can also be performed by attaching the detecting apparatus 1 on the outer side of the arm. As mentioned above, the portion to be detected in the step S10 (the inner side of arm or buttock) for obtaining the pigment index of the body is different from the portion to be detected in the step S40 (the outer side of arm) for obtaining the first redness index. This is because the outer side of arm is more frequently placed under the sunlight than the inner side of arm or buttock, so that the outer side of arm is suitable for detecting the redness index to evaluate the sunburn condition. Besides, the outer side of arm is frequently exposed to the sunlight, so this portion usually has more melanin deposition. In general, different bodies may have different melanin depositions in this portion. Accordingly, if the step S10 is to detect the pigment index with regard to the outer side of arm, the different melanin depositions may cause the detection error and thus overrate the calibrated minimal erythema dose.

After attaching the detecting apparatus 1 on the skin, the NIR light source 151 of the detection light emitting unit 15 outputs an NIR light, which is scattered by the skin area 2 and then received by the light sensing device 12. After receiving the scattered light signal, the light sensing device 12 transmits the light signal to the processing unit 13, and the processing unit 13 calculates to obtain the first redness index of the skin area 2. Besides, the blood vessels in the subcutaneous tissue under the skin area 2 will expand after being irradiated by UV light, so the blood flow in this region increases (congestive reaction), which means that more red blood cells flow through this area. Furthermore, the hemoglobin carried by the red blood cells has an absorption peak in wavelength of the NIR light, so the amount of the hemoglobin in the blood vessels under the skin area 2 will affect the NIR light signal, which is scattered by the skin area 2 and received by the light sensing device 12.

The step S50 is to apply a UV absorption material, which has an SPF (sun protection factor), on the skin area.

In this embodiment, the UV absorption material can be a sunscreen product such as a sunscreen lotion or a foundation product with the SPF of 15, 30 or 50. The user can input the SPF of the UV absorption material to the detecting apparatus 1, so that the processing unit 13 can calculate the maximum UV dose acceptable for the skin applied with the UV absorption material. Then, the protection time can be calculated according to the calculated maximum UV dose and the dose value of the UV light source. The protection time is the period to prevent the skin from sunburn.

The step S60 is to detect a redness situation of the skin area of the body by a second time, after the skin area is irradiated by the UV light from the UV light source, to obtain a second redness index. The second redness index represents the redness index of the skin of the user after exposing to the sunlight (UV light source). According to the detected second redness index, it is possible to determine whether the skin area of the outer side of the arm has sunburn or not. In practice, the detecting apparatus 1 is attached to the skin of the outer side of the arm, the NIR light source 151 of the detection light emitting unit 15 outputs an NIR light, which is scattered by the skin area 2 and then received by the light sensing device 12. After receiving the scattered light signal, the light sensing device 12 transmits the light signal to the processing unit 13, and the processing unit 13 calculates to obtain the second redness index of the skin area 2.

The step S70 is to obtain a first time value if a difference between the first redness index and the second redness index is larger than or equal to the predetermined erythema generation index. Herein, the first time value is the time between the first and second detecting steps.

To perform the step S70, the processing unit 13 of the detecting apparatus 1 retrieves the first and second redness indexes and then compares the first and second redness indexes. As mentioned above, the term "predetermined erythema generation index" is a maximum variation value of the skin redness situation without having sunburn under the irradiation of the sunlight (UV light). Accordingly, if the difference between the first redness index and the second redness index is larger than or equal to the predetermined erythema generation index, it means that the skin area of the body already has sunburn. Then, the processing unit 13 records the time between the first and second detections. Otherwise, if the difference between the first redness index and the second redness index is smaller than the predetermined erythema generation index, it means that the skin area of the body just gets red and does not have sunburn yet. Accordingly, the processing unit 13 does not record the time between the first and second detections. In other words, the second detection is not limited to the second time of performing the detecting step. In this embodiment, the second detection is one of the following detecting step that determines the difference between the first redness index and the second redness index of the current detecting step is larger than or equal to the predetermined erythema generation index. In some cases, the second detection can be the third or fourth time to perform the detecting step. The step S80 is to obtain the minimal erythema dose of the body according the dose value of the UV light source, the SPF of the UV absorption material, and the first time value between the first and second detecting steps. The minimal erythema dose obtained by the step S80 represents the real minimal erythema dose of the detected skin area of the body. Accordingly, the protection time for the user under the sun (UV light) applied with the UV absorption material can be calculated according to the real minimal erythema dose. Thus, the user can precisely determine the time for applying the next sunscreen product (the UV absorption material) to prevent sunburn. More importantly, it is possible to precisely calculate the protection time of different sunscreen products or the maximum acceptable UV dose based on the real minimal erythema dose. In fact, the international standards for SPF have been built several years ago, but the other important factor affecting the sunburn protection, the minimal erythema dose, still has no standards. This invention can provide the real minimal erythema dose so as to complete the skin protection and increase the confidence to the sunscreen products.

Besides, if the step S60 determines that the difference between the first redness index and the second redness index is smaller than the predetermined erythema generation index, which means that the skin area only gets red and has no sunburn, the following steps will be performed.

If the difference between the first redness index and the second redness index is smaller than the predetermined erythema generation index, the step S71 is to continuously irradiate the UV light from the UV light source on the skin area 2 and then to detect a redness situation of the skin area 2 of the body by a third time to obtain a third redness index. If the difference between the first redness index and the third redness index is larger than or equal to the predetermined erythema generation index, the step S72 is to obtain a second time value, which is the time between the first and third detecting steps. Then, the step S84 is to obtain the minimal erythema dose of the body according the dose value of the UV light source, the SPF of the UV absorption material, and the second time value between the first and third detections.

Similarly, if the difference between the first redness index and the third redness index is smaller than the predetermined erythema generation index, this invention can further perform the fourth, fifth or even up to N detecting step until the different between the current detected redness index and the first redness index is larger than or equal to the predetermined erythema generation index, thereby obtaining the desired minimal erythema dose of the body.

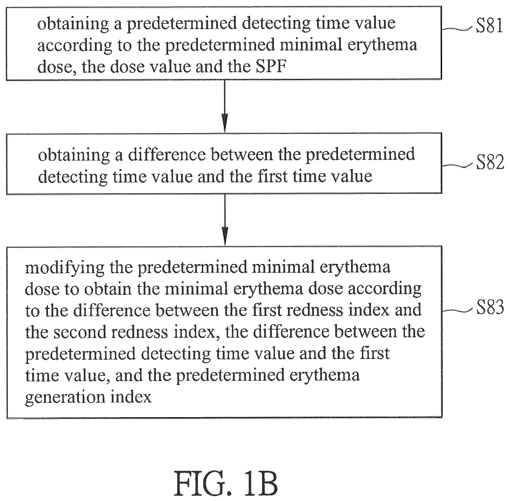

In more detailed, the step S80 includes the following steps S81 to S83. Referring to FIG. 1B, when the detecting apparatus 1 is under operation, the processing unit 13 executes the steps S81 to S83. The step S81 is to obtain a predetermined detecting time value according to the predetermined minimal erythema dose obtained by the step S20, the dose value of the UV light source obtained by the step S30, and the SPF of the UV absorption material obtained by the step S50. The "predetermined detecting time value" represents the predicted maximum time that the user can be exposed to the sunlight (the UV light source) without having sunburn after applying the UV absorption material.

For example, when the step S20 obtains that the pigment index of the user is 10 units, the processing unit 13 accesses the table 1 stored in the storage unit 14 so as to obtain that the corresponding skin level is level 1. Accordingly, the predetermined minimal erythema dose is 15, and the predetermined erythema generation index is 3.

In this case, the step S30 obtains that the dose value of the UV light source is 3 Watt/m$^2$, which means that the UV light dose of the sunlight is 3 J/m$^2$s. Then, the user can input 30 as the SPF of the UV absorption material in the step S50. The definition of SPF is shown as the following equation:

$$SPF = MED_p / MED_u$$

Herein, $MED_u$ represents the minimal erythema dose of the body without applying the sunscreen product, which is the predetermined minimal erythema dose obtained by the step S20. $MED_p$ represents the minimal erythema dose of the body with applying the sunscreen product, which is the maximum time or dose that can cause the minimal erythema on the skin of the body applied with the sunscreen product. Under the same environment, the UV dose value received by the body within a unit time is constant. Accordingly, the unit of the terms "$MED_u$" and "$MED_p$" is time. $MED_p$ is the maximum time that the body applied with the UV absorption material can be protected under the sunlight (UV light) without having sunburn. Based on the above equation, the processing unit 13 can calculate to obtain that the predetermined detecting time is 450 minutes (in step S81). That is, the step S81 determines that the maximum time, which the body applied with the UV absorption material can be protected under the sunlight (UV light) without having sunburn, is 450 minutes. In more detailed, when the user is applied with the sunscreen product with SPF 30, the skin of the user can stay under the sunlight for 450 minutes and does not have sunburn.

The step S82 is to obtain a difference between the predetermined detecting time value and the first time value. The step S83 is to modify the predetermined minimal erythema dose to obtain the calibrated minimal erythema dose according to the difference between the first redness index and the second redness index (obtained in step S70), the difference between the predetermined detecting time value (obtained in step S82) and the first time value, and the predetermined erythema generation index (obtained in step S20).

For example, for the same skin sample and the same environment, the increasing redness of the skin caused by the UV light is in positive correlation to the exposure time. If the detected first redness index is 3 and the detected second redness index is 7, the difference between the first and second redness indexes obtained in step S70 is 4. In this case, the predetermined erythema generation index obtained in step S20 is 3, and the predetermined detecting time value is 450 minutes. The step S70 obtains that the first time value between the first and second detections is 420 minutes. Accordingly, in the step S82, the processing unit calculates to obtain that the difference between the predetermined detecting time value and the first time value obtained in step S70 is 30 minutes. According to the above information, the real minimal erythema dose calibrated by interpolation is 12, which is different from the predetermined minimal erythema dose (15).

In addition, the invention also discloses a detecting device that can be used to execute the above-mentioned method for detecting a minimal erythema dose. As shown in FIG. 3, the detection apparatus 1 includes a UV detection device 11, a light sensing device 12, one or more processing units 13, a storage unit 14, and a detection light emitting unit 15. In this embodiment, the detecting device 1 includes one processing unit 13. The processing unit 13 signally connects to the UV detection device 11, the light sensing device 12, the storage unit 14 and the detection light emitting unit 15. Similarly, the detection apparatus 1 is a portable apparatus or a wearable apparatus. Thus, the user can easily carry the detection apparatus 1 for detecting the intensity of the local UV light and detecting the minimal erythema dose (MED) of a body in real time.

Referring to FIG. 1A, the processing unit 13 of the detection apparatus 1 accesses the required instructions, programs and data from the storage unit 14, and then calculates according to the instructions and data so as to control the operations of other units. The storage unit 14 contains one or more instructions. When the processing unit 13 executes the one or more instructions and programs, the processing unit 13 can perform the above steps S10 to S80, which includes: obtaining a pigment index of the body by the light sensing device 12; obtaining a predetermined minimal erythema dose and a predetermined erythema generation index according to the pigment index; obtaining a dose value of a UV light source by the UV detection device 11; detecting a redness situation of a skin area of the body by a first time to obtain a first redness index by the light sensing device 12; obtaining an SPF (sun protection factor) of a UV absorption material, which is applied on the skin area; after the UV light source irradiates a UV light on the skin area, detecting a redness situation of the skin area of the body by a second time to obtain a second redness index by the light sensing device 12; if a difference between the first redness index and the second redness index is larger than or equal to the predetermined erythema generation index, obtaining a first time value, which is the time between the first and second detecting steps; and obtaining the minimal erythema dose of the body according the dose value, the SPF and the first time value.

The technical contents and details of the steps performed by the detection apparatus 1 are mostly the same as those of the above-mentioned method for detecting a minimal erythema dose of a body, so the detailed descriptions thereof will be omitted.

In summary, the method and apparatus of the present invention can calibrate the MED of a body by detecting the UV dose in the environment, measuring the variation of the redness of skin and the time before and after receiving the UV light, and obtaining the SPF of the UV absorption material. The detecting apparatus of the invention is easily operated to perform the above steps. After obtaining the actual MED of the user, the maximum UV dose or the maximum exposure time under the sunlight after applying the UV absorption material can be calculated according to the SPF of the UV absorption material and the actual MED. Besides, the detecting apparatus of the invention can further prompt the user to apply the UV absorption material again at the right time so as to prevent the damage of skin (e.g. sunburns).

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

**Claims**

1. A method for detecting a minimal erythema dose of a body, comprising following steps of:

   detecting a pigment index of the body;
   obtaining a predetermined minimal erythema dose and a predetermined erythema generation index according to the pigment index;
   obtaining a dose value of a UV light source;
   detecting a redness situation of a skin area of the body by a first time to obtain a first redness index;
   applying a UV absorption material, which has an SPF (sun protection factor), on the skin area;
   irradiating a UV light from the UV light source on the skin area and then detecting a redness situation of the skin area of the body by a second time to obtain a second redness index;
   if a difference between the first redness index and the second redness index is larger than or equal to the predetermined erythema generation index, obtaining a first time value, which is the time between the first and second detecting steps; and
   obtaining the minimal erythema dose of the body according the dose value, the SPF and the first time value.

2. The method according to claim 1, further comprising steps of:

   obtaining a predetermined detecting time value according to the predetermined minimal erythema dose, the dose value and the SPF;
   obtaining a difference between the predetermined detecting time value and the first time value; and
   modifying the predetermined minimal erythema dose to obtain the minimal erythema dose according to the difference between the first redness index and the second redness index, the difference between the predetermined detecting time value and the first time value, and the predetermined erythema generation index.

3. The method according to claim 1, wherein the predetermined minimal erythema dose and/or the predetermined erythema generation index has a positive correlation to the pigment index.

4. The method according to claim 1, further comprising steps of:

   if the difference between the first redness index and the second redness index is smaller than the predetermined erythema generation index, continuously irradiating the UV light from the UV light source on the skin area and then detecting a redness situation of the skin area of the body by a third time to obtain a third redness index;
   if a difference between the first redness index and the third redness index is larger than or equal to the predetermined erythema generation index, obtaining a second time value, which is the time between the first and third detecting steps; and
   obtaining the minimal erythema dose of the body according the dose value, the SPF and the second time value.

5. The method according to claim 1, wherein the UV absorption material is a sunscreen lotion or a foundation product.

**6.** A detecting apparatus for detecting a minimal erythema dose of a body, comprising:

a UV detection device;
a light sensing device;
one or more processing units signally connecting to the UV detection device and the light sensing device; and
a storage unit signally connecting to the one or more processing units, wherein the storage unit stores one or more programs, and when the one or more processing units execute the one or more programs, the one or more processing units perform following steps of:
obtaining a pigment index of the body by the light sensing device,
obtaining a predetermined minimal erythema dose and a predetermined erythema generation index according to the pigment index,
obtaining a dose value of a UV light source by the UV detection device,
detecting a redness situation of a skin area of the body by a first time to obtain a first redness index by the light sensing device,
obtaining an SPF (sun protection factor) of a UV absorption material, which is applied on the skin area,
after the UV light source irradiates a UV light on the skin area, detecting a redness situation of the skin area of the body by a second time to obtain a second redness index by the light sensing device,
if a difference between the first redness index and the second redness index is larger than or equal to the predetermined erythema generation index, obtaining a first time value, which is the time between the first and second detecting steps, and
obtaining the minimal erythema dose of the body according the dose value, the SPF and the first time value.

**7.** The detecting apparatus according to claim 6, wherein the one or more processing units further perform steps of:

obtaining a predetermined detecting time value according to the predetermined minimal erythema dose, the dose value and the SPF;
obtaining a difference between the predetermined detecting time value and the first time value; and
modifying the predetermined minimal erythema dose to obtain the minimal erythema dose according to the difference between the first redness index and the second redness index, the difference between the predetermined detecting time value and the first time value, and the predetermined erythema generation index.

**8.** The detecting apparatus according to claim 6, wherein the predetermined minimal erythema dose and/or the predetermined erythema generation index has a positive correlation to the pigment index.

**9.** The detecting apparatus according to claim 6, wherein the one or more processing units further perform steps of:

if the difference between the first redness index and the second redness index is smaller than the predetermined erythema generation index, after the UV light source continuously irradiates the UV light on the skin area, detecting a redness situation of the skin area of the body by a third time to obtain a third redness index;
if a difference between the first redness index and the third redness index is larger than or equal to the predetermined erythema generation index, obtaining a second time value, which is the time between the first and third detecting steps; and
obtaining the minimal erythema dose of the body according the dose value, the SPF and the second time value.

**10.** The detecting apparatus according to claim 6, wherein the UV absorption material is a sunscreen lotion or a foundation product.

**11.** The detecting apparatus according to claim 6, wherein the detecting apparatus is a portable detecting apparatus.

**12.** The detecting apparatus according to claim 6, further comprising:
an NIR (near infrared) light source for emitting an NIR light, wherein the light sensing device detects the NIR light scattered from the skin area so as to obtain the first redness index or the second redness index.

**13.** The detecting apparatus according to claim 12, further comprising:
a red light source for emitting a red light, wherein the light sensing device detects the red light scattered from the skin area so as to obtain the first redness index or the second redness index.

**14.** The detecting apparatus according to claim 13, further comprising:

a green light source for emitting a green light, wherein the light sensing device detects the green light reflected from the skin area so as to obtain the pigment index.

15. The detecting apparatus according to claim 14, wherein the detecting apparatus comprises two green light sources.

16. The detecting apparatus according to claim 15, wherein the positions of the two green light sources are perpendicular to each other.

17. The detecting apparatus according to claim 16, wherein the NIR light source, the red light source and the green light sources are disposed around the light sensing device.

detecting a pigment index of a body ——S10

obtaining a predetermined minimal erythema dose and a predetermined erythema generation index according to the pigment index ——S20

obtaining a dose value of a UV light source ——S30

detecting a redness situation of a skin area of the body by a first time to obtain a first redness index ——S40

applying a UV absorption material, which has an SPF (sun protection factor), on the skin area ——S50

irradiating a UV light from the UV light source on the skin area and then detecting a redness situation of the skin area of the body by a second time to obtain a second redness index ——S60

if a difference between the first redness index and the second redness index is larger than or equal to the predetermined erythema generation index, obtaining a first time value, which is the time between the first and second detecting steps ——S70

obtaining the minimal erythema dose of the body according the dose value, the SPF and the first time value ——S80

# FIG. 1A

obtaining a predetermined detecting time value according to the predetermined minimal erythema dose, the dose value and the SPF — S81

obtaining a difference between the predetermined detecting time value and the first time value — S82

modifying the predetermined minimal erythema dose to obtain the minimal erythema dose according to the difference between the first redness index and the second redness index, the difference between the predetermined detecting time value and the first time value, and the predetermined erythema generation index — S83

FIG. 1B

if the difference between the first redness index and the second redness index is smaller than the predetermined erythema generation index, continuously irradiating the UV light from the UV light source on the skin area and then detecting a redness situation of the skin area of the body by a third time to obtain a third redness index — S71

if a difference between the first redness index and the third redness index is larger than or equal to the predetermined erythema generation index, obtaining a second time value, which is the time between the first and third detecting steps — S73

obtaining the minimal erythema dose of the body according the dose value, the SPF and the second time value — S80a

## FIG. 1C

FIG. 2A

FIG. 2B

FIG. 2C

FIG. 3A

FIG. 3B

<table>
<tr><td colspan="2"><h1>INTERNATIONAL SEARCH REPORT</h1></td><td>International application No.<br><br>**PCT/CN2017/081187**</td></tr>
</table>

**A. CLASSIFICATION OF SUBJECT MATTER**

A61B 5/00 (2006.01) i; A61B 5/103 (2006.01) i; G01N 21/33 (2006.01) i

According to International Patent Classification (IPC) or to both national classification and IPC

**B. FIELDS SEARCHED**

Minimum documentation searched (classification system followed by classification symbols)

A61B, G01N 21, G06F

Documentation searched other than minimum documentation to the extent that such documents are included in the fields searched

Electronic data base consulted during the international search (name of data base and, where practicable, search terms used)

CNABS, CNTXT, CNKI, EPODOC, WPI, IEEE, TWABS: complexion, ruddy, sunscreen, correct, skin, color+, chroma+, florid, red, minim+, erythema+, dose, MED, ultraviolet, sun, protect+, screen, light, detect+, sens+, time, modif+, adjust+, regulat+

**C. DOCUMENTS CONSIDERED TO BE RELEVANT**

| Category* | Citation of document, with indication, where appropriate, of the relevant passages | Relevant to claim No. |
|---|---|---|
| A | WO 2015172266 A1 (CHEN, Weiyu), 19 November 2015 (19.11.2015), description, page 5, last paragraph to page 17, paragraph 2, and figures 1-12 | 1-17 |
| A | CN 101793824 A (TOPPOLY OPTOELECTRONICS CORP.), 04 August 2010 (04.08.2010), the whole document | 1-17 |
| A | CN 104374872 A (BEIJING UNION MEDICAL COLLEGE HOSPITAL, CHINESE ACADEMY OF MEDICAL SCIENCES et al.), 25 February 2015 (25.02.2015), the whole document | 1-17 |
| A | US 2002022868 A1 (LENDERINK, E. et al.), 21 February 2002 (21.02.2002), the whole document | 1-17 |
| A | US 4423736 A (PURDUE RESEARCH FOUNDATION), 03 January 1984 (03.01.1984), the whole document | 1-17 |
| A | WO 03030707 A2 (FLYBY S.R.L. et al.), 17 April 2003 (17.04.2003), the whole document | 1-17 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 July 2017 (14.07.2017) | **21 July 2017 (21.07.2017)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**WEI, Na**<br><br>Telephone No.: (86-10) **62089912** |

Form PCT/ISA/210 (second sheet) (July 2009)

## INTERNATIONAL SEARCH REPORT
### Information on patent family members

| | International application No. |
|---|---|
| | **PCT/CN2017/081187** |

| Patent Documents referred in the Report | Publication Date | Patent Family | Publication Date |
|---|---|---|---|
| WO 2015172266 A1 | 19 November 2015 | US 2017071528 A1 | 16 March 2017 |
| | | CN 105893721 A | 24 August 2016 |
| CN 101793824 A | 04 August 2010 | TW 201030330 A | 16 August 2010 |
| | | US 2010198026 A1 | 05 August 2010 |
| | | TW I402496 B | 21 July 2013 |
| CN 104374872 A | 25 February 2015 | CN 104374872 B | 20 April 2016 |
| US 2002022868 A1 | 21 February 2002 | EP 1170035 A3 | 10 September 2003 |
| | | US 6736832 B2 | 18 May 2004 |
| | | DE 60114719 T2 | 20 July 2006 |
| | | EP 1170035 B1 | 09 November 2005 |
| | | DE 60114719 D1 | 15 December 2005 |
| | | EP 1170035 A2 | 09 January 2002 |
| US 4423736 A | 03 January 1984 | None | |
| WO 03030707 A2 | 17 April 2003 | EP 1434520 A2 | 07 July 2004 |
| | | AU 2002349818 A1 | 22 April 2003 |
| | | IT FI20010181 A1 | 07 April 2003 |
| | | IT 1327800 B | 30 May 2005 |

Form PCT/ISA/210 (patent family annex) (July 2009)